(19) Europäisches Patentamt
European Patent Office
Office européen des brevets

(11) **EP 2 465 409 A1**

(12) **EUROPEAN PATENT APPLICATION**

(43) Date of publication:
**20.06.2012 Bulletin 2012/25**

(51) Int Cl.:
**A61B 1/00** (2006.01)　　　**A61B 1/05** (2006.01)
**A61B 1/06** (2006.01)

(21) Application number: **11187517.5**

(22) Date of filing: **02.11.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **15.12.2010 JP 2010279288**

(71) Applicant: **Fujifilm Corporation
Minato-ku
Tokyo (JP)**

(72) Inventor: **Kaku, Toshihiko
Kanagawa (JP)**

(74) Representative: **Klunker . Schmitt-Nilson . Hirsch
Destouchesstraße 68
80796 München (DE)**

(54) **Endoscopy system**

(57)　An endoscopy system includes a lighting section for emitting blue narrow-band light, green band light and red band light, an endoscope using an image pickup device for splitting light into blue, green and red spectral bands for photometry, and a processing section for generating a special light inspection image and a normal light inspection image from images produced by the image pickup device.

## FIG. 2

## Description

BACKGROUND OF THE INVENTION

The present invention relates to endoscopy systems allowing both a normal light inspection with white light and a special light inspection with a specified narrow-band light.

Recently employed is an endoscopy system capable of performing the so-called special light inspection in which a mucosal tissue of the living body is illuminated with light in a specified, narrow wavelength band (narrow-band light) as an inspection light to obtain information on the tissue at a desired depth.

It is readily possible in such a special light inspection as above to visualize biological information unobtainable from conventional inspection images, such as a surface microstructure of neovascularity occurring in a mucosal layer or submucosa, and the exaggeration of a lesion. If a carcinomatous lesion site is to be inspected, for instance, states of microvessels or microstructures in a superficial layer of a mucosal tissue are observed in more detail by illuminating the tissue with a blue narrow-band light suitable for the inspection of a superficial tissue layer and a green narrow-band light suitable for the inspection of intermediate and superficial tissue layers, leading to a more accurate diagnosis of the lesion.

Such an endoscopy system as disclosed in JP 3559755 B or JP 3607857 B is known as capable of performing both a normal light inspection and a special light inspection. In the endoscopy system as disclosed in either reference above, a lighting section which has a light source emitting white light and a rotating filter including a red filter for converting white light into red light, a green filter for converting white light into green light, and a blue filter for converting white light into blue light, and an endoscope which produces images with an image pickup device for measuring incident light without splitting the light into its spectral bands are used to carry out so-called frame-sequential imaging.

In the lighting section of the above system, red, green, and blue lights are generated by sequentially inserting the red, green, and blue filters into the optical path of white light through the rotation of the rotating filter, and the lights in different colors are sequentially supplied to the endoscope as inspection lights.

In the endoscope, the inspection lights (red, green, and blue lights) as supplied from the lighting section are propagated, and emitted from the tip (endoscopic portion) so as to illuminate a site to be inspected with the lights, and sequentially produce red, green, and blue images of the site to be inspected (frame-sequentially measure red, green, and blue lights (image with frame-sequential red, green, and blue lights)) by means of the image pickup device. Eventually, the endoscope prepares a color image of the site to be inspected from the individual red, green and blue component images.

The rotating filter of the lighting section has a dual structure with inner and outer parts with respect to the axis of rotation.

In the outer part, red, green, and blue filters each generating light in a typical band are arranged in the direction of rotation as a first filter set having spectral characteristics enabling a spontaneous color reproduction, with the colors of adjacent filters overlapping each other in wavelength region.

In the inner part, red, green, and blue filters each generating light in a narrow band are arranged in the direction of rotation as a second filter set having discrete spectral characteristics in narrow bands, with the colors of the filters being separated from one another in wavelength region.

If a normal light inspection is to be conducted on the above endoscopy system, the first filter set is caused to act on the optical path so as to carry out color-sequential imaging with red, green, and blue components of white light.

If a special light inspection is to be conducted, the second filter set is caused to act on the optical path by displacing the axis of rotation of the rotating filter so as to carry out imaging with narrow-band lights.

The endoscopy system as described above makes it possible to conduct a normal light inspection and a special light inspection on one and the same endoscopy system.

It is often seen in an endoscopic diagnosis that the endoscope tip is inserted deep into the living body, then a normal light inspection (associated with screening with a normal light) is conducted while the endoscope is drawn out, and, if a site suspected to be a lesion is found out, the normal light inspection is replaced by a more detailed inspection with a special light.

In the case where switching between a normal light inspection and a special light inspection is to be carried out by change of filters including those of a rotating type as described above, however, time is required for the change of filters (a time lag is caused by the change of filters). In consequence, a doctor must stand by during the switching between the inspections while keeping conditions unchanged.

A comparison between a normal light inspection image and a special light inspection image may optionally be wanted. It, however, is not possible on a system involving the change of filters to obtain a normal light inspection image and a special light inspection image at a time.

A normal light inspection image and a special light inspection image of one and the same site to be inspected can be obtained indeed by changing filters while making the endoscope immobile. A subject of inspection, however, may move during the time lag even if the endoscope is made immobile. In that case, the target for inspection may move out of the imaging range of the endoscope (frame-out of the target), or fall out of focus.

In order to avoid such inconveniences, a series of images may be stored for later regeneration and comparison, but with unacceptably increased time and effort for diagnosis.

SUMMARY OF THE INVENTION

An object of the present invention is to solve the above problems with the prior art by providing an endoscopy system which allows, on the basis of the configuration of a common endoscopy system, a normal light inspection image and a special light inspection image to be obtained at a time with no time lag being caused by the switching between a normal light inspection and a special light inspection.

In order to achieve the above object, the present invention provides an endoscopy system comprising:

a lighting section having a light emitting unit for emitting blue narrow-band light, green band light and red band light, and a light quantity adjuster for independently adjusting a light quantity of at least one of the blue narrow-band light, the green band light and the red band light;

an endoscope having an illumination unit for propagating light emitted from the lighting section and performing illumination with the light propagated, and an image pickup device for splitting an image of a site to be inspected into blue, green and red spectral bands to perform photometry; and

a processing section for processing an image produced by the endoscope, so as to generate one or both of a normal light inspection image obtained with white light and a special light inspection image obtained with a specified narrow-band light.

Preferably, said green band light is narrow-band light.

Preferably, said light emitting unit comprises a light source emitting white light, an optical divider for dividing the white light emitted from the light source into first, second and third lights, a blue filter for making the first light into the blue narrow-band light, a green filter for making the second light into the green band light, and a red filter for making the third light into the red band light.

Preferably, said light quantity adjuster adjusts the light quantity of at least one of said blue narrow-band light, the green band light and the red band light so that the light quantity of the blue narrow-band light may be higher than that of the green band light and the light quantity of the green band light may be higher than that of the red band light.

Preferably, said processing section generates said special light inspection image from blue and green images produced by the image pickup device of said endoscope.

Preferably, said processing section generates said special light inspection image using a corrected blue image provided by processing of said blue image with said green image and a corrected green image provided by processing of said green image with a red image.

Preferably, said red image is also used for the processing of said blue image in order to provide said corrected blue image.

Preferably, said processing section generates said normal light inspection image from blue, green and red images produced by the image pickup device of said endoscope.

Preferably, said processing section subjects blue, green and red images produced by the image pickup device of said endoscope to gain adjustment in accordance with the light quantity of each light that is given by said light quantity adjuster, and generates said normal light inspection image using the blue, green and red images as subjected to gain adjustment.

Preferably, the endoscopy system further comprises a display unit for displaying an image generated by said processing section.

Preferably, the system comprises two or more display units, with said special light inspection image being displayed on at least one display unit and said normal light inspection image on at least one other display unit.

Preferably, said processing section comprises a control unit for controlling image display on said display unit; display modes are programmed in the processing section that are at least two of a display mode in which only the normal light inspection image is displayed on the display unit, a display mode in which only the special light inspection image is displayed on the display unit, a display mode in which both the normal light inspection image and the special light inspection image are displayed on the display unit in their entireties, a display mode in which both the normal light inspection image and the special light inspection image are displayed on the display unit, with a displayed region being variable, and a display mode in which the normal light inspection image and the special light inspection image are displayed in a switchable manner; and

the system further comprises a selector for the programmed display modes.

The endoscopy system of the present invention uses blue narrow-band light, green (narrow-)band light and red band light as inspection lights, and performs imaging by means of an image pickup device for splitting incident light into blue, green and red lights to measure them.

Consequently, according to the present invention, an inspection image produced by the imaging with a pseudo-white light, namely, a normal light inspection image is obtained using the results of measurement of the blue, green and red lights by the image pickup device. On the other hand, an inspection image produced by the imaging with blue and green narrow-band lights as special lights, namely, a special light inspection image is obtained using the results of measurement of the blue and green lights out of the results of measurement of the blue, green and red lights by the image pickup device.

Thus, the endoscopy system of the present invention allows a normal light inspection image and a special light inspection image to be obtained at a time from one image produced, with no time lag due to inspection switching.

BRIEF DESCRIPTION OF THE DRAWINGS

In the accompanying drawings:

Fig. 1 is a schematic representation of an example of the endoscopy system of the present invention;
Fig. 2 is a diagram schematically showing the configuration of the endoscopy system as shown in Fig. 1;
Fig. 3 is a graph schematically showing exemplary color filter characteristics of an image pickup device in an endoscope; and
Fig. 4 is a block diagram schematically showing a signal processing system of the endoscopy system as shown in Fig. 1.

DETAILED DESCRIPTION OF THE INVENTION

In the following, the endoscopy system according to the present invention is detailed in reference to the preferred embodiment as shown in the accompanying drawings.

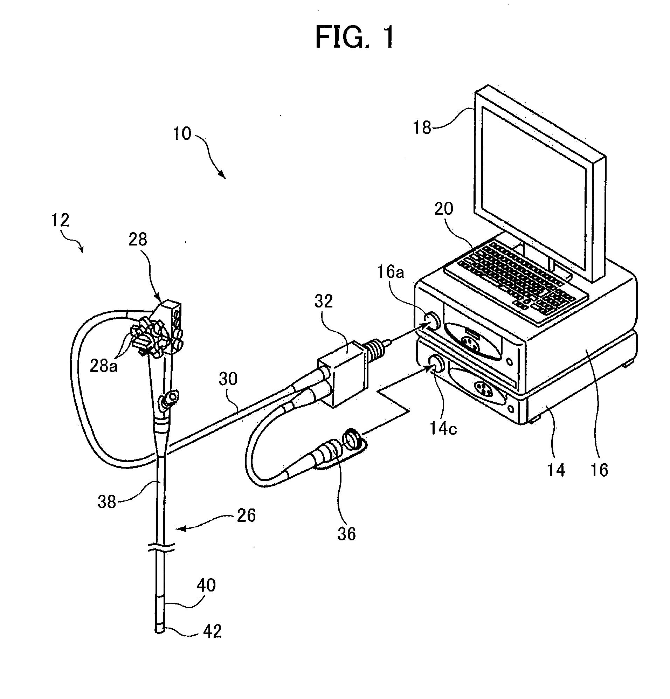

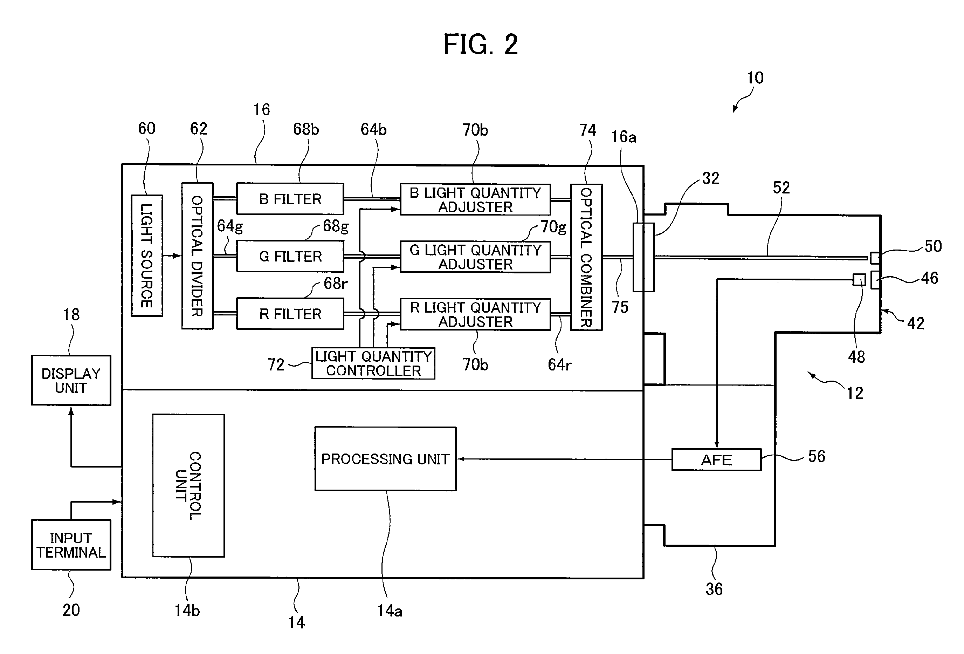

Fig. 1 is a schematic, perspective view of an example of the endoscopy system of the invention, and Fig. 2 schematically shows the configuration of the system as shown in Fig. 1.

An endoscopy system 10 shown includes, for instance, an endoscope 12, a processing section 14 performing processing of an image produced by the endoscope 12, and so forth, and a lighting section 16 for supplying an inspection light (illumination light) for the inspection (imaging) with the endoscope 12. The processing section 14 has a display unit 18 for displaying an image produced by the endoscope 12, and an input terminal 20 for inputting various instructions or the like (the display unit 18 and the input terminal 20 being connected to the unit 14).

The endoscopy system 10 of the present invention may further include a printer (recording apparatus) for outputting an image produced by the endoscope 12 as a hard copy.

As shown in Fig. 1, the endoscope 12 is an electronic endoscope using an image pickup device such as a CCD sensor 48 to produce an image photoelectrically. Similar to the conventional endoscope, the endoscope 12 has an insertion section 26, a manipulation section 38, a universal cord 30, a connector 32, and a video connector 36.

During a normal inspection (diagnosis), the video connector 36 and the connector 32 of the endoscope 12 are joined to a connector socket 14c of the processing section 14 and a connector socket 16a of the lighting section 16, respectively. To the connector 32, a means for performing suction or air supply on a site to be inspected, a water absorbing means for spraying water onto a site to be inspected, and so forth are connected, as is the case with the conventional endoscope.

The insertion section 26 of the endoscope 12 has an elongated flexible portion 38 toward its proximal end, an endoscopic portion (endoscope tip) 42 at its distal end, in which the CCD sensor 48 and so forth are placed, and a bendable portion (angling portion) 40 between the flexible portion 38 and the endoscopic portion 42, and on the manipulation section 28, manipulator knobs 28a for bending the bendable portion 40, and so forth are mounted, as again is the case with the conventional endoscope.

In the endoscopic portion 42, an imaging lens 46, the CCD sensor (image pickup device) 48, a lens for illumination 50, an optical fiber 52, a cover glass (not shown) for the protection of the lenses, and so forth are placed, as schematically shown in Fig. 2.

Some channels and ports, such as a forceps channel and a forceps port for the insertion of various treatment tools including a forceps, and an air/water supply channel and an air/water supply port for suction, air/water supply or the like, are also provided in the endoscope 12, although not shown.

A forceps channel extends through the bendable portion 40 and the flexible portion 38, and communicates with a forceps insertion port provided in the manipulation section 28. An air/water supply channel extends through the bendable portion 40, the flexible portion 38, the manipulation section 28 and the universal cord 30, and communicates with a junction of the connector 32 with a suction, air supply or water supply means.

The optical fiber 52 extends in the endoscope 12 through the bendable portion 40, the flexible portion 38, the manipulation section 28 and the universal cord 30 to the connector 32 to be connected to the lighting section 16.

An inspection light emitted from the lighting section 16 described later enters into the optical fiber 52 through the connector 32, and is propagated through the optical fiber 52. In the endoscopic portion 42, the inspection light as emitted from the tip of the optical fiber 52 is incident on the lens for illumination 50, which illuminates a site to be inspected with the inspection light.

The imaging lens 46 forms an image of the site to be inspected, which has been illuminated with the inspection light, on a light receiving face of the CCD sensor 48.

In the present invention, the CCD sensor 48 as used in the endoscope 12 is a color CCD sensor provided with a blue, green or red filter for each pixel, and adapted to split incident light into blue, green and red lights to measure them. In other words, the CCD sensor 48 as used in the endoscope 12 of the inventive endoscopy system 10 is not a so-called frame-sequential monochrome sensor for sequentially measuring blue, green and red lights without splitting of incident light into its spectral bands, but a simultaneous color sensor for splitting incident light into blue, green and red lights to measure them at a time.

The image pickup device to be used in the present invention is not limited to the CCD sensor 48. Various image pickup devices including a CMOS image sensor are available as long as they are each a color sensor for splitting incident light into blue, green and red lights to measure them at a time.

An output signal from the CCD sensor 48 is transmitted by a signal line, from the endoscopic portion 42 to the video connector 36 through the bendable portion 40, the flexible portion 38, the manipulation section 28, the universal cord 30, and the connector 32.

In the embodiment as shown, an analog front end (AFE) board 56 is placed in the video connector 36.

The AFE board 56 has, for instance, a correlated double sampling circuit, an amplifier (automatic gain control circuit), and an A/D converter. In the AFE board 56, the output signal from the CCD sensor 48 is subjected to noise elimination by correlated double sampling, amplified by the amplifier, then converted by the A/D converter from an analog signal to a digital one so as to output it to the processing section 14 (to a digital signal processor 76 thereof described later) as a digital image signal.

In the endoscopy system of the present invention, such processing as above may be performed not in the video connector 36 but in the connector 32, or in the processing section 14.

As mentioned before, in the endoscopy system 10, the connector 32 of the endoscope 12 is joined to the connector socket 16a of the lighting section 16.

The lighting section 16 is adapted to supply the endoscope 12 with an inspection light for the inspection inside the living body. The inspection light as supplied by the lighting section 16 to the endoscope 12 enters into the optical fiber 52 through the connector 32, and is propagated to the endoscopic portion 42, where the lens for illumination 50 illuminates a site to be inspected with the inspection light as emitted from the tip of the optical fiber 52, as described before.

As schematically shown in Fig. 2, the lighting section 16 of the endoscopy system 10 includes a light source 60, an optical divider 62, a B fiber 64b, a G fiber 64g and an R fiber 64r, a B filter 68b, a G filter 68g and an R filter 68r, a B light quantity adjuster 70b, a G light quantity adjuster 70g and an R light quantity adjuster 70r, a light quantity controller 72, an optical combiner 74, an optical fiber 75, as well as the connector socket 16a as above.

The light source 60 is a light source emitting the light to be used as an inspection light.

In the present invention, various light sources including a xenon lamp and a natural light LED, which are capable of emitting white light, and are used for conventional endoscopy systems and so forth, are usable as the light source 60.

The light as emitted from the light source 60 is divided by the optical divider 62 into three light beams, which are caused to enter into the B fiber 64b, the G fiber 64g and the R fiber 64r as optical fibers, respectively, and propagated toward the optical combiner 74.

The optical divider 62 is a known optical divider (optical coupler).

Between the optical divider 62 and the optical combiner 74, the B filter 68b and the B light quantity adjuster 70b are linked through the B fiber 64b, the G filter 68g and the G light quantity adjuster 70g are linked through the G fiber 64g, as well as the R filter 68r and the R light quantity adjuster 70r are linked through the R fiber 64r.

The entry/emission of light between each optical fiber and the associated filter and light quantity adjuster may be carried out by any known method.

The B filter 68b is a filter for converting the white light as emitted from the light source 60, divided by the optical divider 62, and propagated through the B fiber 64b into blue narrow-band light.

The blue narrow-band light is blue light in a narrow wavelength range, typically light with a band width of not more than 75 nm within a wavelength range of 380 to 480 nm. Specific examples include various blue narrow-band lights used for a so-called special light inspection with an endoscope. More specific examples include light in a wavelength range of 390 to 445 nm, especially light at a wavelength of 400 $\pm$ 10 nm with a center wavelength of 405 nm.

The G filter 68g is a filter for converting the white light as emitted from the light source 60, divided by the optical divider 62, and propagated through the G fiber 64g into green narrow-band light.

The green narrow-band light is green light in a narrow wavelength range, typically light with a band width of not more than 75 nm within a wavelength range of 480 to 580 nm. Specific examples include various green narrow-band lights used for a so-called special light inspection with an endoscope. More specific examples include light in a wavelength range of 530 to 550 nm.

The R filter 68r is a filter for converting the white light as emitted from the light source 60, divided by the optical divider 62, and propagated through the R fiber 64r into red band light.

The red band light is so-called red light, in a wavelength range of 580 to 680 nm for instance. Specifically, the red band light is the light in a common red spectral band that is split off during imaging by a color endoscope.

In the present invention, green light is preferably a narrow-band light as is the case with the embodiment as shown, although not limited to narrow-band lights. Green light may also be green band light. In addition, red light may be red narrow-band light (light in a wavelength range of not more than 75 nm) like the blue and green lights as above.

With respect to the spectral distribution of blue, green and red lights, blue, green and red spectral bands may be separated from one another (the three bands may be discrete), or overlap one another.

The B light quantity adjuster 70b, the G light quantity adjuster 70g, and the R light quantity adjuster 70r adjust the light quantity (light intensity) of blue narrow-band light, green narrow-band light and red band light propagated through the associated optical fibers, respectively.

The B light quantity adjuster 70b, the G light quantity adjuster 70g, and the R light quantity adjuster 70r are not particularly limited, and various known light quantity adjusters, such as an adjustable diaphragm and a gray filter with its density varying sequentially or intermittently, are available.

In other words, various light quantity adjusters are usable in the present invention as long as they are each capable of independently adjusting the light quantity of at least one out of, preferably all of, the blue narrow-band light, the green narrow-band light and the red band light.

Light quantity adjustment by the B light quantity adjuster 70b, the G light quantity adjuster 70g and the R light quantity adjuster 70r is controlled by the light quantity controller 72.

The manner in which the light quantity controller 72 controls the light quantity adjustment is not particularly limited but may be determined as appropriate to the characteristics of the individual color filters, the characteristics of the light source 60, the spectral sensitivity characteristics of the CCD sensor (image pickup device) 48, and the like. For instance, the light quantity adjustment by the individual light quantity adjusters may be controlled so that the blue narrow-band light, the green narrow-band light, and the red band light may be identical to one another in light quantity.

In the present invention, it is preferable that the light quantity controller 72 controls the light quantity adjustment by the individual light quantity adjusters so that the light quantity of the blue narrow-band light may be higher than that of the green narrow-band light (blue light > green light) and, at the same time, the light quantity of the green narrow-band light may be higher than that of the red band light (green light > red light).

As described before, the endoscopy system 10 of the present invention uses blue narrow-band light, green narrow-band light, and red band light as inspection lights, illuminates a site to be inspected with the three inspection lights at a time, and images the site with the CCD sensor 48 for splitting incident light into blue, green and red lights to measure them.

In addition, as detailed later, the endoscopy system 10 (the processing section 14) generates a normal light inspection image from the blue, green and red images as produced by the CCD sensor 48 of the endoscope 12, and a special light inspection image from the blue and green images as produced by the CCD sensor 48.

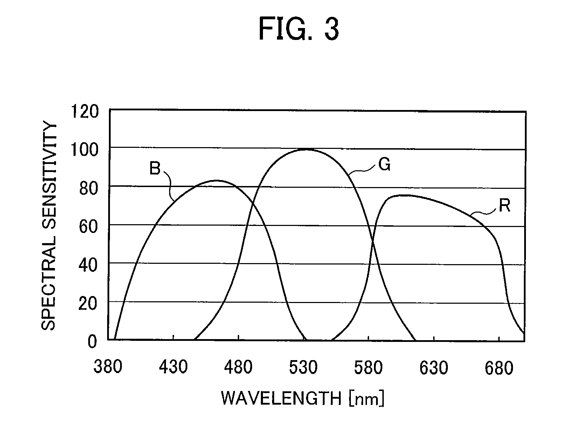

As schematically shown in Fig. 3, the CCD sensor 48 often has such filter characteristics for blue, green and red colors (color filter characteristics) that any of blue, green and red pixels thereof has a sensitivity covering even the spectral band of an adjacent color.

To be more specific: Not only green narrow-band light but red band light (or part thereof) is incident on green pixels where the lights are measured. On the other hand, not only blue narrow-band light but green narrow-band light (or part thereof) is incident on blue pixels where the lights are measured.

If the light quantity of blue narrow-band light is higher than that of green narrow-band light, the blue narrow-band light incident on blue pixels of the CCD sensor 48 will be predominant over the green narrow-band light incident on the blue pixels. Similarly, if the light quantity of green narrow-band light is higher than that of red band light, the green narrow-band light incident on green pixels of the CCD sensor 48 will be predominant over the red band light incident on the green pixels.

Such a configuration (effecting states of blue light > green light and green light > red light) makes it possible to generate a normal light inspection image and a special light inspection image correctly from the images as read by the CCD sensor 48. Moreover, the influence of an error, if any, in the calculation by the processing section 14 as described later can be reduced.

In the present invention, no particular limitation is put on the light quantity ratio between blue narrow-band light and green narrow-band light, or between green narrow-band light and red band light.

In other words, light quantity ratios between two different color lights may be selected as appropriate to the color filter characteristics (spectral sensitivity characteristics) of the CCD sensor 48, the spectral characteristics of the blue, green and red filters of the lighting section 16, the spectral characteristics of the light source 60, and the like so that the effects as above may be achieved.

While a light quantity adjuster used in the present invention is preferably capable of adjusting the light quantity of all the blue narrow-band light, green narrow-band light (green band light) and red band light, the effects as above can be achieved if the light quantity adjuster is capable of adjusting the light quantity of at least one out of the three lights.

In the endoscopy system of the present invention, a light emitting unit for emitting blue narrow-band light, green narrow-band light, and red band light is not limited to the unit of the shown embodiment that consists of the light source 60 emitting white light, the optical divider 62, and the filters. Light emitting units of different structures are available as long as they are each capable of emitting desired blue narrow-band light, green (narrow-)band light and red band light.

For instance, a light emitting unit may be constructed using multiple LEDs, LDs, laser light sources of various types, or other light sources each capable of emitting blue narrow-band light, green (narrow-)band light or red band light (capable of emitting light in a given spectral band).

In that case, adjustment of the output from each light source, use of such a light quantity adjuster as shown in Fig. 2, or a combination thereof may be employed so as to adjust the light quantity of each light to attain the states of blue light > green light and green light > red light as described before.

The blue narrow-band light, green narrow-band light and red band light as adjusted in light quantity by the B light quantity adjuster 70b, the G light quantity adjuster 70g and the R light quantity adjuster 70r, respectively, are combined together by the optical combiner 74 into one light beam, which is propagated through the optical fiber 75 to the connector socket 16a.

The light as delivered to the connector socket 16a of the lighting section 16 is then delivered to the connector 32 of the endoscope 12, and enters from the connector 32 into the optical fiber 52 to be propagated therethrough. The light is eventually used as an inspection light to illuminate a site to be inspected with the inspection light emitted from the endoscopic portion 42 of the endoscope 12.

The optical combiner 74 is a known optical combiner for combining a plurality of light beams into one light beam.

The site as illuminated with the inspection light is imaged by the CCD sensor 48. An image produced by the CCD sensor 48 (output signal from the CCD sensor 48) is subjected to A/D conversion and other processing by the AFE board 56, and supplied to the processing section 14 as a digital image signal (image data/image information).

The processing section 14 is adapted not only to control the endoscopy system 10 as a whole but subject the image signal as supplied (outputted) from the endoscope 12 to specified processing so as to display the processed signal on the display unit 18 as an image produced by the endoscope 12. The processing section 14 includes an image signal processing unit 14a, and a control unit 14b for controlling the processing section 14 as well as the endoscopy system 10 as a whole.

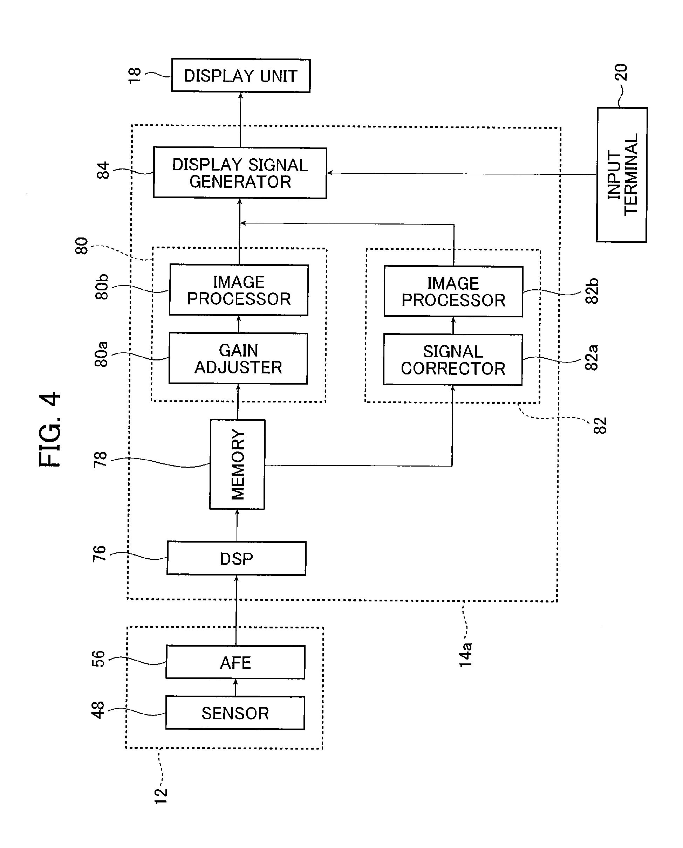

Fig. 4 is a block diagram schematically showing the image signal processing unit 14a of the processing section 14.

As shown in Fig. 4, the processing unit 14a has a digital signal processor (DSP) 76, a memory 78, a normal light image generator 80, a special light image generator, 82, and a display signal generator 84.

In the processing section 14, the image signals (blue image signal, green image signal and red image signal) as captured by the CCD sensor 48 and processed by the AFE board 56 are initially subjected to specified processing such as gamma correction and color correction by the DSP 76, then stored in the memory 78.

Once the image signals have been stored in the memory 78, the normal light image generator 80 reads the blue, green and red image signals from the memory 78 to generate a normal light inspection image, while the special light image generator 82 reads the blue and green image signals from the memory 78 to generate a special light inspection image.

If it was instructed in advance via the input terminal 20 or the like that a normal light inspection image or a special light inspection image is solely to be generated (displayed), only the image generator which has taken instructions to generate an image reads the image signals from the memory 78 to subject them to the processing as described later.

As described before, in the endoscopy system 10, the blue narrow-band light, green narrow-band light and red band light as supplied from the lighting section 16 are used as inspection lights to illuminate a site to be inspected with the three inspection lights at a time. The site thus illuminated is imaged by the CCD sensor 48 for splitting incident light into blue, green and red lights to measure them.

In other words, in the endoscopy system 10 of the present invention, a pseudo-white light provided by merging blue narrow-band light, green narrow-band light and red band light together is uses as an inspection light, and a site to be inspected is imaged by the CCD sensor 48 as a color CCD sensor for measuring blue light, green light and red light (blue, green and red light components) of incident light at a time.

Consequently, an image for a normal light inspection using white light, namely normal light, as an inspection light can be generated by using the blue image signal, green image signal and red image signal as measured by the CCD sensor 48 to generate the image to be displayed.

Since the blue light and the green light as inspection lights are narrow-band lights in their respective colors, an image for a special light inspection (narrow-band light inspection) using blue narrow-band light and green narrow-band light as inspection lights can be generated by using the blue image signal and green image signal as measured by the CCD sensor 48 to generate the image to be displayed. In this regard, the green light is not limited to a narrow-band light, as mentioned before. Generation of a special light inspection image is also possible with green band light.

According to the endoscopy system of the present invention, a normal light inspection image and a special light inspection image are allowed, on the basis of the configuration of a common endoscopy system, to be obtained at a time from one and the same image produced, with no time lag being caused by the switching between a normal light inspection and a special light inspection.

The normal light image generator 80 has a gain adjuster 80a and an image processor 80b.

The gain adjuster 80a preferably performs gain adjustment on the blue, green and red image signals as read from the memory 78 to obtain an image signal similar to that obtained during the inspection with an ordinary white light.

As described before, the inspection lights to be emitted from the lighting section 16 are such that the light quantity of the blue narrow-band light is higher than that of the green narrow-band light and, at the same time, the light quantity of the green narrow-band light is higher than that of the red band light. In other words, the inspection lights with which a site to be inspected is illuminated are in a state of blue light > green light > red light with respect to the light quantity.

The gain adjuster 80a performs gain adjustment of blue, green and red image signals, such as amplification of green and red image signals and reduction of blue and green image signals, to thereby make an image signal similar to that obtained if imaging is carried out using a white inspection light containing blue, green and red lights equal to one another in light quantity.

The method for gain adjustment is not particularly limited, and various methods are applicable as long as they are each capable of compensating for light quantity differences between blue, green and red inspection lights to make an image signal (image produced by the CCD sensor 48) similar to that obtained if imaging is carried out using blue, green and red inspection lights in a uniform light quantity.

In an exemplary method, correction coefficients are provided in accordance with the light quantity difference (light quantity ratio) between blue and green lights as well as the light quantity difference between green and red lights so that the individual light quantity differences may be compensated for, and multiplied by or added to, or again, divided into or subtracted from each image signal. The method, in which each image signal is processed using LUTs provided in accordance with the light quantity difference between blue and green lights as well as the light quantity difference between green and red lights so that the individual light quantity differences may be compensated for, is also applicable.

The image processor 80b performs color conversion processing by 3 x 3 matrix processing, gray scale conversion processing, three-dimensional LUT processing, or the like; color enhancement processing for enhancing the colors of the blood vessel and the mucosa in an image with respect to the average color of the image so that the color difference between the blood vessel and the mucosa may be increased, and the blood vessel may be visible, accordingly; image structure enhancement processing such as sharpness processing and edge emphasis; and so forth on the image signals as subjected to gain adjustment, so as to obtain an image signal for a normal light inspection image and supply the signal to the display signal generator 84.

The special light image generator 82 has a signal corrector 82a and an image processor 82b.

The signal corrector 82a preferably processes a green image signal with a red image signal to obtain a corrected green image signal, and processes a blue image signal with a green image signal to obtain a corrected blue image signal.

In the endoscopy system 10 of the present invention, a special light inspection image may be generated using a blue image signal and a green image signal as such.

During the imaging with the CCD sensor 48, however, not only green narrow-band light but red band light is incident on green pixels where the lights are measured, while not only blue narrow-band light but green narrow-band light is incident on blue pixels where the lights are measured, attributed to the color filter characteristics of the sensor 48 as described before. Consequently, if a special light inspection image is generated using a blue image signal and a green image signal as such, a blue image will be affected by a green image component, and a green image will be affected by a red image component.

It is thus preferable that a green image signal is processed with a red image signal so as to remove a red image signal component from the green image signal, and a blue image signal is processed with a green image signal so as to remove a green image signal component from the blue image signal.

The red image signal to be used for the processing of a green image signal and the green image signal to be used for the processing of a blue image signal may be the image signals for the red and green pixels, respectively, each of which pixels should be a subpixel constituting one pixel along with the pixel at which processing is to be performed. It is also possible to appropriately select a pixel adjacent to the pixel at which processing is to be performed, so as to employ the image signal for the selected pixel.

Correction to a green image signal is made by the following equation, for instance:

```
     (corrected green image signal) = (green image signal) - α *

 (red image signal) [i.e., (corrected green pixel) = (green

 pixel) - α * (red pixel)].
```

In the equation, $\alpha$ is a coefficient for obtaining a red light component measured at a green pixel. A coefficient allowing the calculation of a red light component measured at a green pixel may be selected as appropriate to the color filter characteristics of the CCD sensor 48 and the like.

Correction to a blue image signal is made by the following equation, for instance:

```
     (corrected blue image signal) = (blue image signal) - β *

 (corrected green image signal) [i.e., (corrected blue pixel) =

 (blue pixel) - β * (corrected green pixel)].
```

The following equation is also usable:

```
     (corrected blue image signal) = (blue image signal) - β

 [(green image signal) - α * (red image signal)].
```

In the equations, $\beta$ is a coefficient for obtaining a green light component measured at a blue pixel. Similar to the above, a coefficient allowing the calculation of a green light component measured at a blue pixel may be selected as appropriate to the color filter characteristics of the CCD sensor 48 and the like.

The image processor 82b processes a corrected blue image signal and a corrected green image signal to obtain an image signal for a special light inspection image.

In an image displayed, one pixel is composed of three subpixels in blue, green, and red, while an image signal for a special light inspection image only includes a corrected blue image signal (blue pixel) and a corrected green image signal (green pixel). The image processor 82b multiplies a corrected green image signal by a specified coefficient to allocate the signal to a red pixel of an image displayed and, at the same time, multiplies a corrected blue image signal by a specified coefficient to allocate the signal to green and blue pixels of the image, with the two corrected image signals being thus adapted for three subpixels in blue, green and red of the image.

Subsequently, the image processor 82b performs color conversion processing by 3 x 3 matrix processing, gray scale conversion processing, three-dimensional LUT processing, or the like; color enhancement processing for enhancing the colors of the blood vessel and the mucosa in an image with respect to the average color of the image so that the color difference between the blood vessel and the mucosa may be increased, and the blood vessel may be visible, accordingly; image structure enhancement processing such as sharpness processing and edge emphasis; and so forth on the individual image signals so as to obtain an image signal for a special light inspection image and supply the signal to the display signal generator 84.

The display signal generator 84 performs necessary processing such as color space conversion on the supplied image signals for a normal light inspection image and a special light inspection image so as to make them into image signals adapted for the display on the display unit 18.

In the endoscopy system 10, at least two display modes are programmed out of, for instance, the display mode in which only normal light inspection images are to be displayed; the display mode in which only special light inspection images are to be displayed; the display mode in which a normal light inspection image and a special light inspection image are displayed side by side within one screen of the display unit 18 in their entireties (the images may be the same, different, or adjustable in size); the display mode in which a normal light inspection image and a special light inspection image are displayed side by side beyond one screen of the display unit 18, with a displayed region being variable with a slider bar, a trackball, or the like; and the display mode in which either a normal light inspection image or a special light inspection image is displayed at a time, with the switching between the images being carried out in accordance with the switching instruction from a switching means provided on the input terminal 20 and/or the manipulation section 28 of the endoscope 12.

The display modes are selectable/specifiable by a selecting means provided on the input terminal 20 and/or the manipulation section 28 of the endoscope 12.

The display signal generator 84 performs image enlargement/reduction, image layout, or even incorporation of character information, such as the name of the subject, in accordance with the selected display mode so as to generate an image signal for display, and cause a corresponding image to be displayed on the display unit 18.

If two or more display units 18 are used, the display signal generator 84 may generate image signals so that a normal light inspection image may be displayed on one display unit 18, and a special light inspection image may be displayed on another display unit 18.

If three or more display units 18 are used, a normal light inspection image may be displayed on one display unit 18 and a special light inspection image on another as mentioned above, and, on a yet another display unit 18, image display may be carried out in accordance with any of the display modes as above.

In the following, exemplary functions of the endoscopy system 10 are described.

If the start of imaging with the endoscope 12 is instructed via the input terminal 20, the light source 60 of the lighting section 16 comes on, the individual light quantity adjusters are adjusted by the light quantity controller 72, and the CCD sensor 48 begins imaging (photometry).

The light as emitted from the light source 60 is divided by the optical divider 62 into three light beams, which are processed by the B, G and R filters to obtain blue narrow-band light, green narrow-band light and red band light, respectively, and the lights thus obtained are adjusted in light quantity by the corresponding light quantity adjusters so as to attain a state of blue light > green light > red light.

The blue narrow-band light, green narrow-band light and red band light as subjected to light quantity adjustment are combined together by the optical combiner 74 into one light beam, which is supplied as an inspection light to the connector 32 of the endoscope 12 through the connector socket 16a.

The inspection light as supplied to the connector 32 of the endoscope 12 is propagated through the optical fiber 52 to the endoscopic portion 42 and, in the endoscopic portion, emitted from the tip of the optical fiber 52. The lens for illumination 50 then illuminates a site to be inspected (the inside of the living body) with the inspection light.

An image of the site to be inspected which has been illuminated with the inspection light is formed on the light receiving face of the CCD sensor 48 by the imaging lens 46, so that the CCD sensor 48 performs imaging (photometry).

An output signal from the CCD sensor 48 is supplied to the AFE board 56. The AFE board 56 subjects the output signal from the CCD sensor 48 to noise elimination by correlated double sampling, amplification, A/D conversion, and so forth, so as to supply it to the DSP 76 of the processing section 14 (the image signal processing unit 14a) as a digital image signal.

The DSP 76 subjects the image signal to specified processing such as gamma correction and color correction, and stores the processed image signal in the memory 78.

From the memory 78 with image signals stored therein, the normal light image generator 80 reads blue, green and red image signals, while the special light image generator 82 reads blue and green image signals.

In the normal light image generator 80, the gain adjuster 80a subjects the read image signal to gain adjustment so as to make an image similar to that produced if imaging is carried out using white light containing blue, green and red lights equal to one another in light quantity, as described before. In addition, the image processor 80b performs color conversion processing, color enhancement processing, and image structure enhancement processing on the image signal as subjected to gain adjustment, so as to obtain an image signal for a normal light inspection image and supply the signal to the display signal generator 84.

On the other hand, in the special light image generator 82, the signal corrector 82a corrects the read green image signal with a red image signal to obtain a corrected green image signal, and corrects the read blue image signal with a green image signal to obtain a corrected blue image signal. In addition, the image processor 82b allocates the corrected green image signal to a red pixel of an image displayed and, at the same time, allocates the corrected blue image signal to blue and green pixels of the image to thereby allow a pixel composed of three subpixels, and performs color conversion processing, color enhancement processing, and image structure enhancement processing on the image signals so as to obtain an image signal for a special light inspection image and supply the signal to the display signal generator 84.

The display signal generator 84, as having been supplied with the image signals for a normal light inspection image and for a special light inspection image, generates an image signal for display in accordance with the selected/ specified display mode, in which, for instance, a normal light inspection image and a special light inspection image are to be displayed side by side within one screen of the display unit 18 in their entireties, and causes corresponding images to be displayed on the display unit 18.

The endoscopy system according to the present invention has been detailed in reference to the preferred embodiment, although the present invention is not limited to the above embodiment. Various improvements and modifications may be made within the gist of the present invention.

**Claims**

1. An endoscopy system comprising:

   a lighting section having a light emitting unit for emitting blue narrow-band light, green band light and red band light, and a light quantity adjuster for independently adjusting a light quantity of at least one of the blue narrow-band light, the green band light and the red band light;
   an endoscope having an illumination unit for propagating light emitted from the lighting section and performing illumination with the light propagated, and an image pickup device for splitting an image of a site to be inspected into blue, green and red spectral bands to perform photometry; and
   a processing section for processing an image produced by the endoscope, so as to generate one or both of a normal light inspection image obtained with white light and a special light inspection image obtained with a specified narrow-band light.

2. The endoscopy system according to claim 1, wherein said green band light is narrow-band light.

3. The endoscopy system according to claim 1 or 2, wherein said light emitting unit comprises a light source emitting white light, an optical divider for dividing the white light emitted from the light source into first, second and third lights, a blue filter for making the first light into the blue narrow-band light, a green filter for making the second light into the green band light, and a red filter for making the third light into the red band light.

4. The endoscopy system according to any one of claims 1 to 3, wherein said light quantity adjuster adjusts the light quantity of at least one of said blue narrow-band light, the green band light and the red band light so that the light quantity of the blue narrow-band light may be higher than that of the green band light and the light quantity of the green band light may be higher than that of the red band light.

5. The endoscopy system according to any one of claims 1 to 4, wherein said processing section generates said special light inspection image from blue and green images produced by the image pickup device of said endoscope.

6. The endoscopy system according to claim 5, wherein said processing section generates said special light inspection image using a corrected blue image provided by processing of said blue image with said green image and a corrected green image provided by processing of said green image with a red image.

7. The endoscopy system according to claim 6, wherein said red image is also used for the processing of said blue image in order to provide said corrected blue image.

8. The endoscopy system according to any one of claims 1 to 7, wherein said processing section generates said normal light inspection image from blue, green and red images produced by the image pickup device of said endoscope.

9. The endoscopy system according to claim 8, wherein said processing section subjects blue, green and red images produced by the image pickup device of said endoscope to gain adjustment in accordance with the light quantity of each light that is given by said light quantity adjuster, and generates said normal light inspection image using the blue, green and red images as subjected to gain adjustment.

10. The endoscopy system according to any one of claims 1 to 9, further comprising a display unit for displaying an image generated by said processing section.

11. The endoscopy system according to claim 10, wherein the system comprises two or more display units, with said special light inspection image being displayed on at least one display unit and said normal light inspection image on at least one other display unit.

12. The endoscopy system according to claim 10 or 11, wherein:

    said processing section comprises a control unit for controlling image display on said display unit;
    display modes are programmed in the processing section that are at least two of a display mode in which only the normal light inspection image is displayed on the display unit, a display mode in which only the special light inspection image is displayed on the display unit, a display mode in which both the normal light inspection image and the special light inspection image are displayed on the display unit in their entireties, a display mode in which both the normal light inspection image and the special light inspection image are displayed on the display unit, with a displayed region being variable, and a display mode in which the normal light inspection image and the special light inspection image are displayed in a switchable manner; and

the system further comprises a selector for the programmed display modes.

# FIG. 1

FIG. 2

# FIG. 3

# FIG. 4

**Europäisches Patentamt**
**European Patent Office**
**Office européen des brevets**

# EUROPEAN SEARCH REPORT

Application Number

EP 11 18 7517

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/141747 A1 (KUBO MASAHIRO [JP] ET AL) 10 June 2010 (2010-06-10) <br> * paragraphs [0007] - [0012], [0018], [0036] - [0046] * <br> * figures 1-2 * <br> ----- | 1-12 | INV. <br> A61B1/00 <br> A61B1/05 <br> A61B1/06 |
| X | US 2003/013937 A1 (TSUJITA KAZUHIRO [JP] ET AL) 16 January 2003 (2003-01-16) <br> * paragraphs [0011] - [0020], [0109] - [0011] * <br> * figure 7 * <br> ----- | 1-12 | |
| X | EP 1 908 393 A1 (OLYMPUS MEDICAL SYSTEMS CORP [JP]) 9 April 2008 (2008-04-09) <br> * paragraphs [0004] - [0007], [0023], [0063] - [0086] * <br> * figures 6-8 * <br> ----- | 1-12 | |
| X | US 2002/175993 A1 (UENO HITOSHI [JP] ET AL) 28 November 2002 (2002-11-28) <br> * paragraphs [0023] - [0025], [0064] - [0094] * <br> * figures 1-4 * <br> ----- | 1-12 | TECHNICAL FIELDS SEARCHED (IPC) <br><br> A61B |

The present search report has been drawn up for all claims

| Place of search | Date of completion of the search | Examiner |
|---|---|---|
| The Hague | 7 March 2012 | Faymann, Juan |

EPO FORM 1503 03.82 (P04C01)

**ANNEX TO THE EUROPEAN SEARCH REPORT
ON EUROPEAN PATENT APPLICATION NO.**

EP 11 18 7517

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2012

| Patent document cited in search report | | Publication date | Patent family member(s) | | Publication date |
|---|---|---|---|---|---|
| US 2010141747 | A1 | 10-06-2010 | EP | 2196134 A1 | 16-06-2010 |
| | | | JP | 2010136748 A | 24-06-2010 |
| | | | US | 2010141747 A1 | 10-06-2010 |
| US 2003013937 | A1 | 16-01-2003 | JP | 2002345733 A | 03-12-2002 |
| | | | US | 2003013937 A1 | 16-01-2003 |
| EP 1908393 | A1 | 09-04-2008 | CN | 101232840 A | 30-07-2008 |
| | | | EP | 1908393 A1 | 09-04-2008 |
| | | | JP | 4709606 B2 | 22-06-2011 |
| | | | JP | 2007029555 A | 08-02-2007 |
| | | | KR | 20080028449 A | 31-03-2008 |
| | | | TW | I316852 B | 11-11-2009 |
| | | | US | 2010217077 A1 | 26-08-2010 |
| | | | WO | 2007013245 A1 | 01-02-2007 |
| US 2002175993 | A1 | 28-11-2002 | DE | 60224321 T2 | 08-01-2009 |
| | | | EP | 1258221 A2 | 20-11-2002 |
| | | | US | 2002175993 A1 | 28-11-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3559755 B

- JP 3607857 B